# EUROPEAN PATENT SPECIFICATION

(11) **EP 0 888 371 B1**
(45) Date of publication and mention of the grant of the patent: **22.05.2002**
(21) Application number: 97940823.4
(22) Date of filing: 05.09.1997
(51) Int. Cl.: C07H 17/075, C07H 15/203, C12Q 1/34

(54) **4,7-DIALKOXY-N-ACETYLNEURAMINIC ACID DERIVATIVES AND METHODS FOR DETECTION OF INFLUENZA TYPE A AND B VIRUSES IN CLINICAL SPECIMENS**
4,7-DIALKOXY-N-ACETYLNEURAMINSAUREDERIVATE UND METHODEN ZUR ERKENNUNG VON INFLUENZA TYP A UND B IN KLINISCHEN PROBEN
DERIVES D'ACIDE 4,7-DIALCOXY N-ACETYLNEURAMINIQUE ET PROCEDES DE DETECTION DES TYPES A ET B DU VIRUS DE LA GRIPPE DANS DES PRELEVEMENTS CLINIQUES

(30) Priority: 25.09.1996 US 718666
(43) Date of publication of application: 07.01.1999
(73) Proprietor: Oklahoma Medical Research Foundation, Oklahoma City Oklahoma 73104 (US)
(72) Inventor: LIAV, Avraham, Denver, CO 80224 (US); HANSJERGEN, Joyce Anne, Oklahoma City, OK 73116 (US); SHIMASAKI, Craig, David, Oklahoma City, OK 73134 (US)
(74) Representative: Hansen, Bernd, Dr. Dipl.-Chem.
(86) International application number: PCT/US97/15602
(87) International publication number: WO 98/13372

(56) References cited:
- WO-A-91/09971
- WO-A-91/09972
- WO-A-91/10744
- US-A- 5 252 458

## Description

The present invention provides chromogenic and fluorogenic 4,7-dialkoxy-N-acetylneuraminic acid substrates which can be used to detect influenza types A and B in clinical samples or specimens. More particularly, the present invention provides 4,7-dialkoxy-N-acetylneuraminic acid substrates which can be used to distinguish between various viruses having neuraminidase reactivity. Thus, influenza type A and B viruses can be distinguished from parainfluenza type 1, 2, 3, and 4 and mumps viruses using the 4,7-dialkoxy-N-acezylneuraminic acid derivatives of this invention.

### BACKGROUND OF THE INVENTION

Infectious diseases are the single most common reason for physician office visits. Viruses are responsible for more of these infections than all other groups of microorganisms combined. Of all the various infections caused by viruses, the respiratory viruses (influenza A and B; parainfluenza 1, 2, 3, and 4; respiratory syncytial virus; and adenovirus) are the most prevalent as a group. The lethality of the influenza virus was discovered in as early as 430 BC in the plague of Athens (Langmuir et al., New Engl. J. Medicine, 313 (1985) 1027). Influenza is the number one cause of acute respiratory illness and a contributor to the sixth leading cause of death in the United States annually (Monthly Vital Statistics Report, Vol.43, No. 6 (1995)). As a result, the development of diagnostic methods for viruses and viral infections has become increasingly important.

The rapid diagnosis of viral infections has also become an integral part of good medical practice. Some viruses have definable antigens against which antibodies can be produced. Therefore, immunoassays have been widely used for the measurement of the presence of a virion. Where it is desirable to measure a broader group of virions, it may be possible to detect a particular component of the virus. For example, influenza viruses express surface glycoproteins having neuraminidase (sialidase) activity. The neuraminidase enzyme hydrolyzes substrates that contain 2-ketosidically linked N-acetylneuraminic acid (Neu5Ac, also known as sialic acid). Neu5Ac consists of a backbone of nine carbon atoms, a carboxyl group and an N-acetyl group. The general structure, as well as the numbering system used to denote the carbon atoms, is shown below. 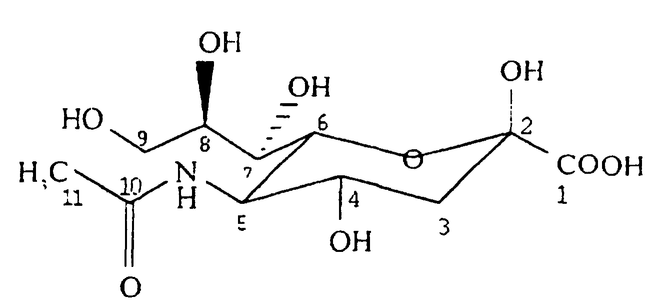 When a virion with neuraminidase activity is incubated with a chromogenic or fluorogenic glycoside of Neu5Ac, the enzyme will cleave the chromogenic or fluorogenic aglycon from the substrate, and the reaction product will indicate the presence of a virion. Throughout this specification, the N-acetyl group attached to the 5-position carbon in the above formula will be denoted as AcHN.

One method for detecting the presence of a virus through the reaction of an enzyme with a chromogenic substrate for the enzyme is described in U.S. Patent No. 5,252,458. An assay for the direct measurement of influenza neuraminidase was developed by Yolken et al. (J. Infectious Diseases, 142 (1980) 516-523). Yolken et al. used the 4-methylumbelliferyl-2-ketoside of Neu5Ac as a fluorescent substrate to measure neuraminidase activity in preparations containing small quantities of cultivated virus as well as in some nasal wash specimens from human volunteers infected with the influenza virus. Yolken et al. suggested that "successful development of fluorometric enzyme assays for the detection of influenza neuraminidase might thus provide for a practical means of influenza diagnosis that is sufficiently rapid to allow for the institution of appropriate preventive and therapeutic interventions." According to Yolken et al., colorimetric assays were insufficiently sensitive for clinical applications. In contrast, Yolken et al. noted that fluorometric assays might be suitable for detecting influenza neuraminidase in clinical samples.

Pachucki et al. (J. Clinical Microbiology, 26 (1986; 2654-2666) tested the 4-methylumbelliferyl-2-ketoside of Neu5Ac on clinical specimens collected from influenza patients. Due to its low sensitivity, the assay was not useful in detecting neuraminidase directly and rapidly in clinical specimens. The assay did, however, identify 91% of virus-positive isolates 25 hours after inoculation of tissue cultures.

The use of modified Neu5Ac substrates can increase the specificity of the neuraminidase assay. In sialic acids, the 4-position carbon (C-4) has been reported to play an important role in enzyme-substrate interactions. Further, since it is known that salivary bacterial enzymes exhibit neuraminidase activity (Varki et al., J. Biol. Chem., 258 (1983) 12465-12471), it is essential to eliminate these undesired interactions. It has already been shown that ketosides of 4-methoxy-Neu5Ac are resistant towards certain bacterial sialidases, but are cleaved rapidly by certain viral sialidases (Beau et al., Eur. J. Biochem., 106 (1980) 531-540).

Chromogenic derivatives of N-acetylneuraminic acid modified in the 4-position are used as substrates to detect influenza infection according to WO-A-9109972.

Although modification of the 4-position of N-acetylneuraminic acids provides specificity between certain viral and certain bacterial neuraminidase reactivity, it would still be desirable to obtain substrates which allow further specificity or differentiation between the various viral neuraminidase reactivities while maintaining the specificity between viral and bacterial neuraminidase reactivities. Such substrates would allow, for example, high specificity for particular types of neuraminidase-containing viruses and allow better and more directed treatment regimes. Such substrates would also allow for more accurate surveillance of viral infections and more focused medical intervention as appropriate. The chromogenic and fluorogenic 4,7-modified N-acetylneuraminic acid substrates of the present invention allows for further specificity or differentiation between the various viral neuraminidase reactivities while maintaining the specificity between viral and bacterial neuraminidase reactivities.

### SUMMARY OF THE INVENTION

This invention relates to chromogenic and fluorogenic 4,7-modified N-acetylneuraminic acid substrates which can be used for the detection and identification of influenza viruses in clinical specimens. More specifically, this invention relates to chromogenic and fluorogenic 4,7-dialkoxy N-acetylneuraminic acid substrates which can be used for the detection and identification of influenza viruses in clinical specimens. These 4,7-modified N-acetylneuraminic acid substrates can be used in diagnostic tests to distinguish between influenza type A and B viruses and other viruses possessing neuraminidase enzymes in clinical specimens. This invention also relates to diagnostic methods employing such substrates.

As used herein the terms "chromogenic or fluorogenic group" and "marker or reporter group" are intended to include, without limitation, molecules that exhibit absorbance or fluorescence. The term "color" is likewise intended to include, without limitation, absorbance and fluorescence.

It is an object of this invention to provide chromogenic and fluorogenic 4,7-modified N-acetylneuraminic acid substrates useful in diagnostic methods for the detection of viruses. Another object of this invention is to provide a practical, convenient, and cost effective method for the detection of influenza type A and B viruses in clinical specimens. Another object of this invention is to provide a practical, convenient, and cost effective diagnostic method which can distinguish between influenza type A and B viruses and other viruses having general neuraminidase reactivity in clinical specimens.

Still another object of this invention is to provide a 4,7-dialkoxy-N-acetylneuraminic acid of the general formula 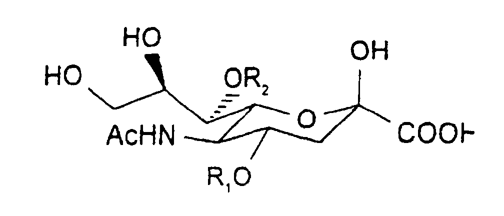 wherein R₁ and R₂ are alkyl groups containing 1 to 4 carbon atoms. Preferably, both R₁ and R₂ are methyl groups.

Still another object of this invention is to provide a 4,7-dialkoxy-N-acetylneuraminic acid substrate of the general formula 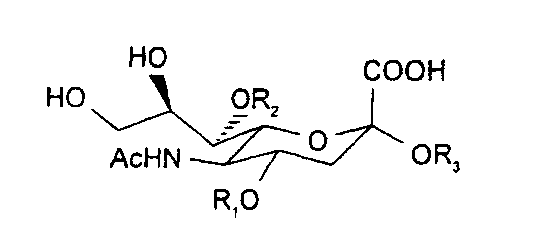 wherein R₁ and R₂ are alkyl groups containing 1 to 4 carbon atoms and R₃ is a chromogenic or fluorogenic group. Preferably, both R₁ and R₂ are methyl groups and R₃ is a chromogenic group.

Still another object of this invention is to provide a method for detecting influenza type A and B viruses in a clinical sample from an individual suspected of having a respiratory viral infection, said method comprising:
(1) incubating the clinical sample with a chromogenic or fluorogenic 4,7-dialkoxy N-acetylneuraminic acid substrate of the general formula 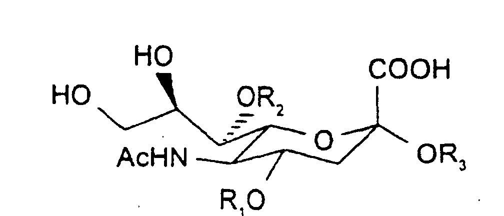 wherein R₁ and R₂ are alkyl radicals containing 1 to 4 carbon atoms and R₃ is a chromogenic or fluorogenic group that exhibits a distinct and characteristic color when cleaved from the substrate or a salt of the substrate;
(2) observing the incubated clinical sample to determine whether the distinct and characteristic color is formed, wherein the formation of the distinct and characteristic color indicates the presence of influenza type A or B viruses in the clinical sample.

Still another object of this invention is to provide a method for detecting influenza type A and B viruses in a clinical sample from an individual suspected of having a respiratory viral infection, said method comprising:
(1) dividing the clinical sample into a first portion and a second portion;
(2) incubating the first portion with a chromogenic or fluorogenic 4,7-dialkoxy N-acetylneuraminic acid first substrate of the general formula 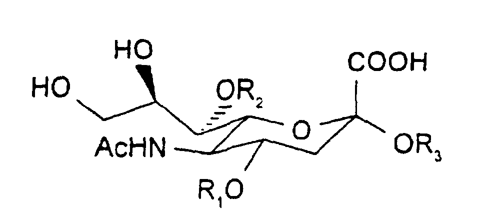 wherein R₁ and R₂ are alkyl radicals containing 1 to 4 carbon atoms and R₃ is a first chromogenic or fluorogenic group that exhibits a distinct and characteristic first color when cleaved from the first substrate or a salt of the first substrate;
(3) observing the incubated first portion to determine whether the distinct and characteristic first color is formed, wherein the formation of the distinct and characteristic first color indicates the presence of influenza type A or B viruses in the clinical sample;
(4) incubating the second portion with a chromogeric or fluorogenic 4-alkoxy N-acetylneuraminic acid second substrate of the general formula 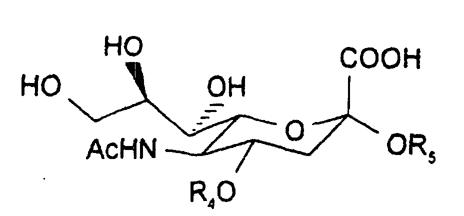 wherein R₄ is an alkyl radical containing 1 to 4 carbon atoms and R₅ is a second chromogenic or fluorogenic group that exhibits a distinct and characteristic second color when cleaved from the second substrate or a salt of the second substrate; and
(5) observing the incubated second portion to determine whether the distinct and characteristic second color is formed, wherein the formation of the distinct and characteristic second color indicates the presence of neuraminidase reactive viruses in the clinical sample;
wherein the presence of the first and second colors indicates the presence of influenza type A or B viruses alone or in combination with neuraminidase reactive viruses other than influenza type A and B viruses in the clinical sample;
wherein the presence of the second color and the absence of the first color indicates neuraminidase reactive viruses other than influenza type A and B viruses in the clinical sample; and
wherein the absence of the first and second colors indicates the absence of neuraminidase reactive viruses in the clinical sample.

Other objects, advantages, features, and characteristics of the present invention will become more apparent upon consideration of the following description and the appended claims.

### DETAILED DESCRIPTION OF THE INVENTION

The present invention relates to a 4,7-dialkoxy-N-acetylneuraminic acid of the general formula 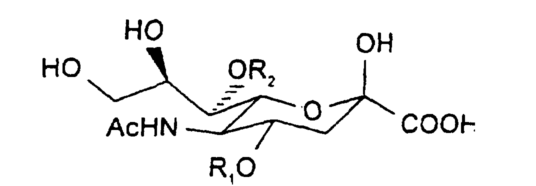 wherein R₁ and R₂ are alkyl groups containing 1 to 4 carbon atoms. R₁ and R₂ may be the same or different alkyl groups. The higher alkyl groups (i.e., when R contains 3 to 4 carbon atoms) can include linear and branched isomers. Preferably R₁ and R₂ are alkyl groups containing 1 or 2 carbons atoms; more preferably R₁ and R₂ are both methyl groups.

The 4,7-dialkoxy-N-acetylneuraminic acid of the present invention can be prepared using the following general reaction scheme:
The starting material 1 (8,9-O-isopropylidine-methyl ester-methyl ketoside derivative of NeuSAc) is prepared from NeuSAc as generally described in our U.S. Patent 5,556,963 issued September 17, 1996 (application Serial Number 08/286,573 filed August 5, 1994), which is hereby incorporated by reference. Neu5Ac is commercially available (MediHerb Inc., 4540 S. Navajo #1, Englewood, Co. 80110). It may also be synthesized enzymatically from N-acetyl-D-mannosamine and pyruvic acid using the 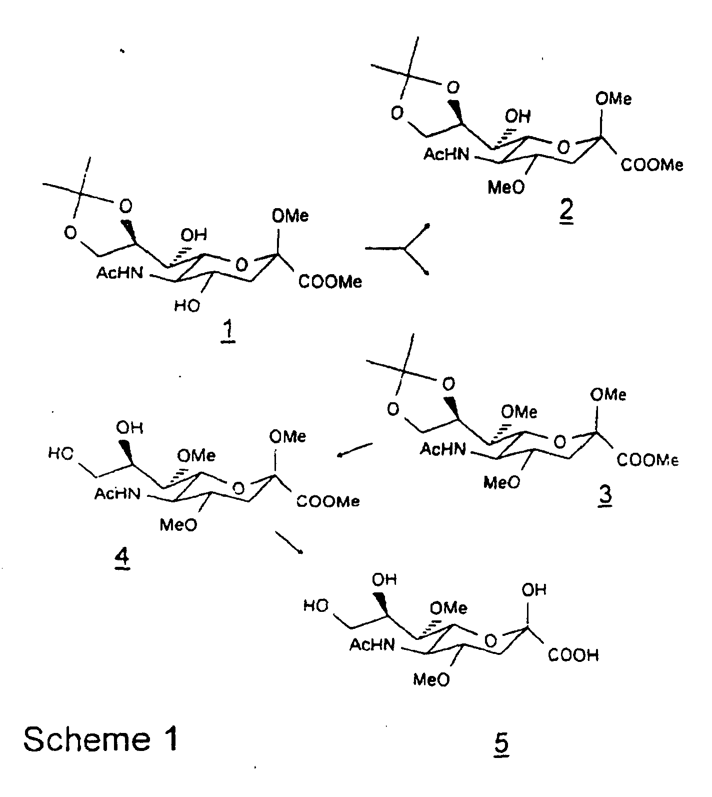 procedure described by Kim et al., J. Am. Chem. Soc., 110 (1988) 6481, and illustrated by the following equation: 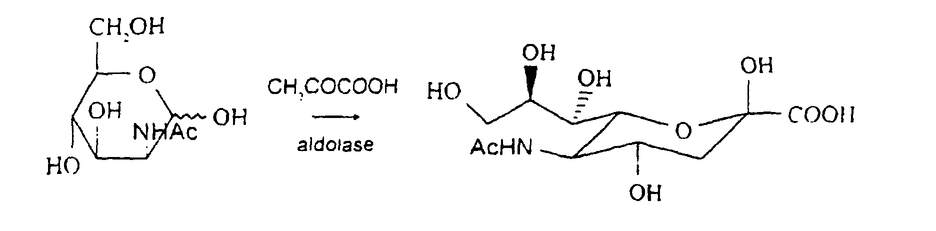 The enzymatic reaction can be monitored by thin layer chromatography (TLC) and the product can be purified by ion exchange chromatography.

Neu5Ac is first converted into an alkyl ester alkyl ketoside of general formula 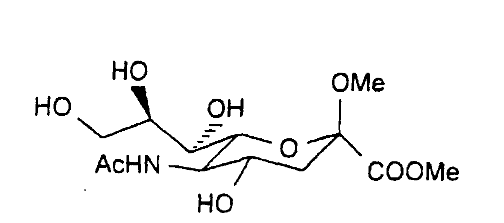 as described in U.S. Patent 5,556,963. The vicinal hydroxyl groups at C-8 and C-9 of this methyl ester methyl ketoside are protected by the formation of a ketal (**1**) by treatment with effective amounts of acetone and an acid catalyst to form the ketal. Suitable acid catalysts include p-toluenesulfonic acid, salts of p-toluenesulfonic acid such as the pyridinium salt (PPTS and other salts, ZnCl₂, FeCl₃, and the like. The preferred acid catalyst is the non-hygroscopic pyridinium salt of p-toluenesulfonic acid.

The protected methyl ester methyl keioside (**1** is then alkylated to form a mixture of compound **2** containing an alkoxy group at the 4-position and compound **3** with alkoxy groups at both the 4- and 7-positions. Alkylation of the hydroxyl group at C-4 and C-7 may be methylation, ethylation, propylation, or butylation, whereby the hydroxy group at C-4 in 2 is converted to a -OR group and, the hydroxy groups at C-4 and C-7 in **3** are converted to -OR groups where R is an alkyl radical containing 1 to 4 carbon atoms. Preferably, the alkylation at C-4 and/or C-7 is methylation or ethylation, whereby 4-methoxy or 4-ethoxy derivatives or 4,7-dimethoxy or 4,7-diethoxy derivatives are obtained. More preferably, the alkylation at C-4 and/or C-7 is methylation, whereby 4-methoxy or 4,7-dimethoxy derivatives are obtained. Introduction of higher alkyl groups at the C-4 and C-7 positions is generally slower than methylation and the yields are somewhat lower. Furthermore, chromogenic substrates with higher alkyl groups tend to be less susceptible to enzymatic cleavage than 4,7-dimethoxy-Neu5Ac, thereby resulting in less sensitive assays. Nonetheless, for some specific applications and assays, such higher alkyl groups at C-4 and C-7 may be useful and even preferred.

Alkylation at the more sterically hindered free hydroxyl group at C-7 can be encouraged by controlling the reaction conditions as described immediately hereafter. In accordance with the process, intermediate **1** is created with an excess (generally greater than about 1.5 molar equivalents) of an alkylating agent in about an 80% dispersion of sodium hydride. The alkylating agent is selected from the group consisting of dimethyl sulfate, diethyl sulfate, dipropyl sulfates, and dibutyl sulfates. The reaction is generally conducted at a temperature of from about 0°C to about 30°C for about 10 minutes to about 48 hours. Preferably the reaction temperature is in the range of about 0°C to about 22°C. Longer reaction times are generally preferred when forming the higher alkoxy groups at the 4- and 7-positions. In a preferred embodiment of the invention, a mechylacion reaction to form methoxy groups at C-4 and C-7 is conducted at a temperature of from about 0°C to about 30°C, more preferably from about 0°C to about 22°C, for about 10 minutes to about 30 minutes. In another preferred embodiment of the invention, an ethylation reaction to form ethoxy groups at C-4 and C-7 is conducted at a temperature of from about 0°C to about 30°C, more preferably from about 0°C to about 22°C, for about 1 hour to about 24 hours.

Treatment of the protected methyl ester methyl ketoside **1** with excess alkylating agent (e.g., dimethyl sulfate) produces a mixture of compound **2** containing an alkoxy group at the 4-position and compound **3** with alkoxy groups at both the 4- and 7-positions. Generally, an excess of about 1.5 molar equivalents of alkylating agent is used. Preferably about 1.5 to about 2.0 molar equivalents of alkylating agent is used. Generally the amount of the desired compound **3** is increased relative to compound **2** as the amount of alkylation agent increases. The reaction mixture resulting from such treatment generally contains the 4-alkoxy compound **2** as the major product and about 10 to 20 weight percent of the 4,7-dialkoxy compound **3.** Partial separation of the compounds **2** and **3** can be obtained, for example, by column chromatography and subsequent crystallization from an acetone-hexane mixture which preferentially removes the 4-alkoxy compound **2.** The resulting residue contains a mixture of the two compounds enriched with the 4,7-dialkoxy material; generally the molar ratio of the two compounds is increased to at least about 1:1.

Removal of the ketal group from compound **3** is achieved by treatment with about 80% acetic acid. Acetic acid hydrolysis can also result in partial acetylation at the C-9 hydroxyl group. Hence, the hydrolysis product can be treated with sodium methoxide to remove any acetate groups on C-9. Final deprotection of **4** is performed by alkaline treatment and subsequent acid hydrolysis to give the final 4,7-dialkoxy-Neu5Ac product (**5**). Of course, the 4-alkoxy compound **2** present in the mixture will also be treated in a similar manner to yield the corresponding 4-alkoxy-Neu5Ac compound.

The resulting 4,7-dialkoxy-Neu5Ac can be further utilized through coupling to any suitable marker or reporter group, including for example, a chromogenic or fluorogenic marker group. The preferred marker or reporter group is a chromogenic group, including, for example, 4-chloro-1-naphthol, 6-bromo-1-naphthol, and 5-bromo-4-chloro-indole. Chromogenic modified 4,7-dialkoxy-Neu5Ac can be incorporated into a neuraminidase assay useful for detecting viral neuraminidase activity from influenza type A and B in clinical samples or specimens. Methods for synthesizing and using such 4,7-position modified chromogenic N-acetylneuraminic acid substrates in viral assays are similar to those described for the related 4-position modified substrates in PCT Publication Number WO 91/09972; Yolken et al., J. Infectious Diseases, 142 (1980) 516-523; and Pachucki et al., J. Clinical Microbiology, 26 (1988) 2664-2666. Of course, the present 4,7-dialkoxy-N-acetylneuraminic acids can be used in other viral assays.

Generally the chromogenic or fluorogenic marker group can be incorporated into the 4,7-dialkoxy-Neu5Ac (**5**) using the following reaction scheme (using 5-bromo-3-indolyl as an example marker group) : 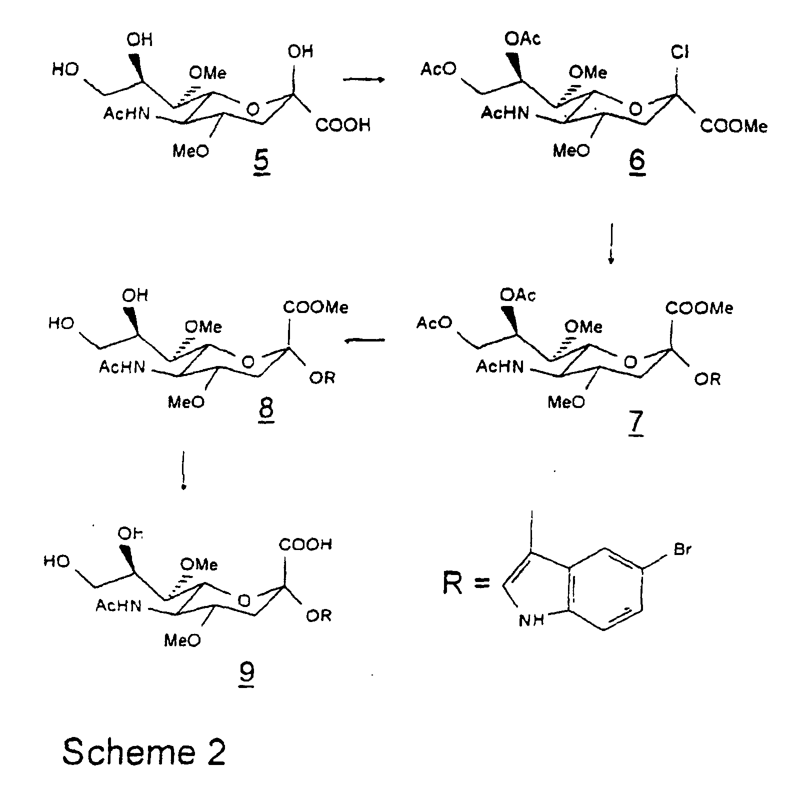 Compound **5** is first converted into the corresponding methyl ester derivative by treatment with concentrated trifluoroacetic acid and methanol, and the ester is then reacted with excess acetyl chloride to form the chloroacetate-methyl ester derivative of 4,7-dialkoxy-Neu5Ac (**6**). Coupling of **6** with the sodium salt of 5-bromo-3-indolyl gives rise to the 5-bromo-indol-3-ol-4,7-dimethoxy-N-acetylneuraminic acid-acetoxy-methyl ester (**7**). De-acetylation of **7** is effected by treatment with sodium methoxide in methanol to yield compound **8** and subsequent treatment with sodium hydroxide gives the sodium salt of 5-bromo-3-indolyl-4,7-dimethoxy-N-acetylneuraminic acid (**9**). Of course, the 4-alkoxy-NeuSAc in the mixture undergoes similar coupling reactions to form the 5-bromo-3-indolyl-4-methoxy-N-acetylneuraminic acid salt.

The coupled 4,7-dialkoxy derivative **9** is then separated from the mixture of the two coupled compounds (i.e., the 4-alkoxy and 4,7-dialkoxy derivatives) by high performance liquid chromatography (HPLC) using, for example, a C18 reverse-plate silica column. The mixture of the coupled products can be dissolved in water and loaded onto the column. The products are separated by an increasing gradient of methanol with the appropriate fractions collected and pooled. The resulting purified 5-bromo-3-indolyl-4,7-dimethoxy-N-acetylneuraminic acid (**9**) can be dried and stored until use. Generally, the resulting product is essentially free of the 4-methoxy derivative. It is important that this mixture be essentially free of the 4-methoxy derivative since it is highly reactive wich mumps virus and other neuraminidase-containing viruses.

As noted above, the chromogenic or fluorogenic 4,7-dialkoxy derivatives of this invention are of the general formula 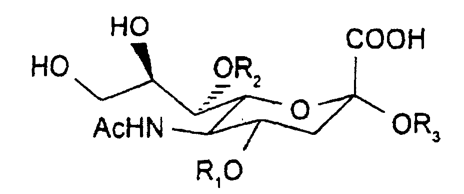 wherein R₁ and R₂ are alkyl groups containing 1 to 4 carbon atoms and R₃ is a chromogenic or fluorogenic group. R₁ and R₂ may be the same or different alkyl groups. Preferably, both R₁ and R₂ are methyl groups and R₃ is a chromogenic group. More preferably, R₃ is 4-methylumbellifery;, 3-cyanoumbelliferyl, 2-nitrophenyl, 4-nitrophenyl, 3-resorufin, 5-bromo-4-chloro-3-indolyl, 5-bromo-3-indolyl, 3-indolyl, nitrophenylazophenyl, nitrophenylazoresorcinyl, 3-methoxyphenyl, 3-dimethylaminophenyl, 4-chloro-1-naphthyl, or 6-bromo-2-naphthyl. Even more preferably, R₃ is 4-methylumbelliferyl, 5-bromo-4-chloro-3-indolyl, or 5-bromo-3-indolyl. The most preferred R₃ is 5-bromo-3-indolyl. Simple salts of these substrates, such as the Na⁺, K⁺, and NH₄⁺ salts, may also be used.

Examples of 4,7-dialkoxy chromogenic Neu5Ac derivatives falling within the above formula include 4-methylumbelliferyl-4,7-dimethoxy-N-acetylneuraminic acid-alpha-ketoside, 2-nitrophenyl-4,7-dimethoxy-N-acetylneuraminic acid-alpha-ketoside, 4-nitrophenyl-4,7-methoxy-N-acetylneuraminic acid-alpha-ketoside, 3-cyanoumbelliferyl-4,7-dimethoxy-N-acetylneuraminic acid-alpha-ketoside, 3-resorufin-4,7-dimethoxy-N-acetylneuraminic acid-alpha-ketoside, 5-bromo-4-chloro-3-indolyl-4,7-dimethoxy-N-acetylneuraminic acid-alpha-ketoside, 5-bromo-3-indolyl-4,7-dimethoxy-N-acecylneuraminic acid-alpha-ketoside, 3-indolyl-4,7-dimethoxy-N-acetylneuraminic acid-alpha-ketoside, 2-[4-(4-nitrophenylazo)phenyl]-4,7-dimethoxy-N-acetylneuraminic acid-alpha-ketoside, 2-[4-(4-nitrophenylazo)resorcinyl]-4,7-dimethoxy-N-acetylneuraminic acid-alpha-ketoside, 3-methoxyphenyl-4,7-dimethoxy-N-acetyl-neuraminic acid-alpha-ketoside, 3-dimethylaminophenyl-4,7-dimethoxy-N-acetylneuraminic acid-alpha-ketoside, 6-bromo-2-naphthyl-4,7-dimethoxy-N-acetylneuraminic acid-alpha-ketoside, 4-chloro-1-naphthyl-4,7-dimethoxy-N-acetylneuraminic acid-alpha-ketoside, as well as the corresponding 4,7-diethoxy, 4,7-dipropyl, and 4,7-dibutyl derivatives. Generally, the 4,7-dimethoxy derivatives are preferred. If desired, "mixed" 4,7-dialkoxy derivatives (e.g., 4-methoxy-7-ethoxy) can be used.

The chromogenic or fluorogenic 4,7-dialkoxy derivatives can be used in diagnostic tests for influenza type A and B viruses in clinical samples. The clinical samples that are tested in the invention will typically be pharyngeal, nasopharyngeal, or respiratory secretions collected from patients suffering, or suspected of suffering, from influenza as wash, swab, or expectorate specimens. The wash, expectorate, or swab will preferably be combined with an aqueous buffer solution containing a stabilizer prior to mixing with the substrate. The buffer solution generally contains a buffer that maintains the pH at about 4 to 7, preferably 5.5 to 6.5, optionally about 0.1% to 10% by weight nonionic detergent, a small amount (1-20 mM) of alkaline earth metal cation (Ca, Mg, preferably Ca), and a sufficient amount of a stabilizer selected from the group consisting of alditols, monosaccharides, and disaccharides to enhance the thermal stability of the neuraminic acid in the sample.

The volume of buffer solution combined with the specimen will normally be 0.1 to 2 ml. The buffer may be organic or inorganic. Suitable buffers include, for example, conventional buffers of organic acids and salts thereof such as citrate buffers (e.g., monosodium citrate-disodium citrate mixture, citric acid-trisodium citrate mixture, citric acid-monosodium citrate mixture, etc.), acetate buffers (e.g., acetic acid-sodium acetate mixture), succinate buffers (e.g., succinic acid-monosodium succinate mixture, succinic acid-sodium hydroxide mixture, succinic acid-disodium succinate mixture, etc.), tartrate buffers (e.g., tartaric acid-tartrate mixture, tartaric acid-potassium tartrate mixture, tartaric acid-sodium hydroxide mixture etc.), fumarate buffers (e.g., fumaric acid-monosodium fumarate mixture, fumaric acid-disodium fumerate mixture, monosodium fumarate acid-disodium fumerate mixture), gluconate buffers (e.g., gluconic acid-sodium gluconate mixture, gluconic acid-sodium hydroxide mixture, gluconic acid-potassium gluconate mixture, etc.) oxalate buffers (e.g., oxalic acid-sodium oxalate mixture, oxalic acid-sodium hydroxide mixture, oxalic acid-potassium oxalate mixture, etc.), lactate buffers (e.g., lactic acid-sodium lactate mixture, lactic acid-sodium hydroxide mixture, lactic acid-potassium lactate-mixture, etc.), acetate buffers (e.g., acetic acid-sodium acetate mixture, acetic acid-sodium hydroxide mixture, etc.), malate buffers (e.g., D,L-malic acid-disodium malate mixture), phosphate buffers (e.g., monosodium phosphate-disodium phosphate mixture, monosodium phosphate-sodium hydroxide mixture, trisodium phosphate-hydrochloric acid mixture, etc.), 2-(N-morpholino)ethanesulfonic acid, [bis-(2-hydroxyethyl)imino]tris(hydroxymethyl)methane, N-2-acetamidoiminodiacetic acid, 1,3-bis[tris(hydroxymethyl)methylamino]propane, piperazine-N,N'-2-echanesulfonic acid), N-2-acetamido-2-aminoechanesulfonic acid, 3-(N-morpholino)-2-hydroxypropanesulfonic acid, 3- (N-morpholino)propanesulfonic acid, 2-[tris(hydroxymethyl)methylamino]ethanesulfonic acid, N-2-hydroxyethylpiperazine-NN'-2-ethanesulfonic acid, 3-[tris-(hydroxymethyl) methylamino]-2-hydroxypropanesulfonic acid.

Non-ionic detergents useful in the buffer solution include, for example, the Pluronics, such as Polysorbate 20 or Polysorbate 80, Triton X-100, NP-40, and alkyl glucosides such as C₈ and C₉ alkyl glucosides. The detergent is an optional component and facilitates release of the neuraminidase from the viral envelope. Stabilizers that are used in the buffer solution include, for example, trihydric or higher alditols, such as glycerin, erythritol, arabitol, xylitol, sorbitol, mannitol, the hexoses glucose and fructose and the disaccharide sucrose. These stabilizers can be used alone or in combination. In order to stabilize the activity of the neuraminidase-containing viruses, the stabilizers are added to the liquid formulation/excipient system in an amount from 0.2 M to 2.1 M and preferably, 0.6 M to 2.0 M. Once mixed with the buffer solution, the sample may be stored for prolonged periods, preferably at 2°C to 8°C, without significant loss of neuraminidase activity.

The substrate will normally be added to the buffered, stabilized sample in amounts ranging between C.05 mM and 0.5 mM. The mixture is incubated at ambient temperature to physiological temperature (i.e., about 18°C to 40°C) for a time sufficient to permit any neuraminidase in the sample to react with the substrate. That time will normally be in the range of 1 minute to 4 hours, usually at 5 to 120 minutes, and more usually 30 to 60 minutes. If there is neuraminidase activity in the sample, the marker or reporter group will be released from the substrate and the liberated marker or reporter precipitate will impart a characteristic color to the mixture. Especially for colorimetric derivatives, the resulting reaction mixture is preferably transferred to a collection device containing a porous membrane filter and an absorbent pad to wick away the solution. Once such collection device is shown in our copending application Serial Number 08/479,789 (filed June 7, 1995), which is hereby incorporated by reference. In the presence of influenza type A and B neuraminidase, the reporter group on the 4,7-modified Neu5Ac is released by the action of neuraminidase and the resulting reporter molecule is collected as a colored precipitate on the porous filter membrane. The presence of such a colored product indicates a positive diagnosis for influenza types A and B: the absence of such a colored product indicates a negative diagnosis for influenza types A and B.
Concentration or collection of the colored precipitate increases the sensitivity of the diagnostic test. Such collection devices are not, however, necessary for the practice of the invention.

The following table indicates the characteristic color generated when neuraminidase reacts with various chromogenic or fluorogenic 4,7-modified Neu5Ac derivatives and releases the reporter molecule.

| Released Reporter Molecule | Type of Detection | Color |
|---|---|---|
| 5-bromo-4-chloro-3-indolol | colorimetric/ visual | blue/purple in the presence of nitroblue tetrazolium |
| 5-bromo-3-indolol | colorimetric/ visual | blue/purple in the presence of nitroblue tetrazolium |
| 3-indolol | colorimetric/ visual | blue/purple in the presence of nitroblue tetrazolium |
| 4-methylumbelliferone | fluorometric | fluorescent emission at 454 nm after excitation at 360 nm |
| 3-cyanoumbelliferone | fluorometric | fluorescent emission at 454 nm after excitation at 415 nm |
| resorufin | colorimetric/ visual | pink/red |
| 2-nitrophenol | colorimetric/ visual | yellow |
| 4-nitrophenol | colorimetric/ visual | yellow |
| nitrophenylazophenol | colorimetric/ visual | orange |
| nitrophenylazoresorcinol | colorimetric/ visual | green blue (presence of Mg⁺⁺) |
| 3-methoxyphenol | colorimetric/ visual | red to blue after reaction with diazonium salt |
| 3-dimethylaminophenol | colorimetric/ visual | red to blue after reaction with diazonium salt |
| 4-chloro-1-naphthol | colorimetric/ visual | red to blue after reaction with diazonium salt |
| 6-bromo-2-naphthol | colorimetric/ visual | red to blue after reaction with diazonium salt |

The present chromogenic and fluorogenic 4,7-dialkoxy Neu5Ac derivatives only exhibit the characteristic color in the presence of influenza type A and B virus. They do not exhibit the characteristic color (i.e., no release of the reporter molecule occurs) in the presence of parainfluenza types 1, 2, 3, and 4, mumps, respiratory syncytial viruses, and/or adenoviruses. Moreover, the chromogenic and fluorogenic 4,7-dialkoxy Neu5Ac derivatives do not exhibit bacterial neuraminidase reactivity. To maintain the desired degree of selectivity, the 4,7-dialkoxy Neu5Ac derivatives should be essentially free of the corresponding 4-alkoxy Neu5Ac derivatives. The maximum levels of the 4-alkoxy Neu5Ac derivatives which allow acceptable selectivity can be determined experimentally using known strains of the various types of viruses. Generally, the levels of 4-alkoxy Neu5Ac derivatives should be less than about 5 weight percent, and preferably less than about 0.5 weight percent.

A potentially more informative diagnostic test can be obtained by combining the neuraminidase reactivity and selectivity of the present chromogenic and fluorogenic 4,7-dialkoxy Neu5Ac derivatives and the chromogenic and fluorogenic 4-alkoxy Neu5Ac derivatives in a single test. In such a system, the clinical sample is divided into two portions which are then incubated separately with the 4,7-dialkoxy and the 4-alkoxy Neu5Ac derivatives, respectively. The presence and/or absence of characteristic color in the two incubated samples can be used to more closely determine the type of virus, if any, present in the clinical sample. If only influenza type A or B viruses are present in the clinical sample, then both the 4-alkoxy and the 4,7-dialkoxy derivatives will exhibit their characteristic colors of their reporter molecules. If both influenza type A or B viruses and neuraminidase reactive viruses other than influenza type A and B viruses are present in the clinical sample, then each portion will exhibit the characteristic color of its reporter molecule. If only neuraminidase reactive viruses other than influenza type A and B viruses are present in the clinical sample, then only the 4-alkoxy derivative will exhibit the characteristic color of its reporter molecule. If no neuraminidase reactive viruses are present in the clinical sample, then neither portion will exhibit the characteristic color of its reporter molecule.

Accordingly, the present invention provides simple and rapid techniques for selectively diagnosing influenza -- especially types A and B -- that may be carried out in the clinic or physician's office and enable the physician to prescribe the appropriate therapy to treat the infection and/or the appropriate prophylactic treatment to persons in close contact with the infected patient.

The following examples are intended to illustrate the invention.

Example 1. The synthesis of 4,7-dimechoxy-Neu5Ac was carried out using the reactions shown in Scheme 1. A cold (ice bath temperature) solution of the isopropylidene derivative **1** (1.2 g) in about 10 ml of dry acetonitrile was saturated with nitrogen. Sodium hydride (270 mg; 80% dispersion in oil) was added and the mixture stirred for 20 minutes. Dimethyl sulfate (1 ml) was added and the stirring continued for an additional 30 minutes using an ice bath to cool the mixture. The resulting mixture was filtered through celite and the precipitate was washed with dry acetonitrile. The filtrate was evaporated and the residue was dried and extracted with acetone. After filtration, the filtrate was again evaporated. The resulting residue was dried and chromatographed on silica gel. Elution with methylene chloride/methanol (25:1) removed several byproducts. Continued elution with the same solvent system provided a syrup containing **2** and **3** which was crystallized from acetone-hexane. Crystalline **2** (177 mg) was collected. The filtrate contained a mixture of **2** and **3** (about 1:1) but with a higher proportion of 3 than the original mixture.

The filtrate containing **3** (1.22 g) was treated with 80% aqueous acetic acid (15 ml) at 85°C for one hour. The mixture was evaporated and co-evaporated with water. The residue was chromatographed. Elution with methylene chloride/methanol (5:1) removed a minor byproduct. Continued elution with the same solvent system gave the partially deprotected product **4** (0.87 g; 80% yield).

The 4,7-dimethoxy-methyl ester methyl ketoside (**4**) (0.87 g) was treated with 1 M sodium hydroxide (3 ml) in methanol (5 ml) and water (5 ml) at room temperature for one hour. The mixture was neutralized with Dowex 50 (H⁺) resin; the resin was removed by filtration and washed with methanol. The combined filtrate was evaporated. The residue was treated with Dowex 50 (H⁺) resin (1.5 g) in 0.025 M hydrochloric acid at 100°C for two hours. The resin was removed by filtration and the filtrate collected and evaporated. The residue was dried under vacuum to give essentially pure 4,7-dimethoxy-Neu5Ac (**5**) (0.71 g; 88% yield).

Example 2. The synthesis of 5-bromo-3-indolyl-4, 7-dimethoxy-Neu5Ac was carried out using the reactions shown in Scheme 2. Compound **5** (1.0 g) was treated with trifluoroacetic acid (0.2 ml) in methanol (25 ml) overnight at room temperature. The mixture was evaporated. The residue was dried and treated with acetyl chloride (5 ml) in methylene chloride (5 ml). The reaction mixture was stirred overnight at room temperature and then evaporated. The residue was dried co provide the crude chloride **6** (1.36 g). The crude product **6** was not further purified because of its instability.

A suspension of the crude product **6** (0.7 g) and 1-acetyl-5-bromo-indol-3-ol (0.26 g) in acetone (5 ml) was saturated with nitrogen. A 1 M sodium hydroxide solution (1 ml) was added. The reaction mixture was stirred under nitrogen for one hour. After evaporation, the residue was dried and chromatographed on silica gel. Elution with methylene chloride/methanol (25:1) removed the unreacted chromogen (0.128 g). Continued elution with the same solvent system gave fractions containing mainly the coupled product **7** (0.114 g). The coupled product **7** (0.114 g) was treated with 1 M sodium methoxide in methanol (0.1 ml) for 30 minutes at room temperature. After neutralization with Dowex 50 (H⁺) resin, the resin was removed and washed with methanol. The combined filtrate was evaporated. The residue was dried and chromatographed on silica gel. Fractions containing mainly the coupled product **8** were pooled and evaporated to give about 60 mg of **8**.

The coupled product **8** (60 mg) was treated with 1 M sodium hydroxide (0.5 ml) in 50% aqueous methanol (5 ml) for 30 minutes at room temperature. The mixture was neutralized with Dowex 50 (H⁺) resin. The resin was removed by filtration and washed with methanol. The combined filtrate was evaporated and the residue was purified by HPLC. The first fractions contained an uncoupled product. The coupled material appeared as a broad peak which consisted of both coupled 4-methoxy-Neu5Ac and coupled 4,7-dimethoxy-Neu5Ac product **9.** Since minor amount of the coupled 4-methoxy derivative could render the diagnostic analysis unspecific, only highly pure fractions containing **9** were pooled. Upon evaporation, highly purified **9** (4 mg) was collected; the collected material contained less than about 5 weight percent of the corresponding 4-alkoxy derivative.

Example 3. This example demonstrates the specificity of the 5-bromo-3-indolyl-4,7-dimethoxy-Neu5Ac (**9**) of Example 2 in diagnostic tests for influenza A and B in cultured viruses. Viruses were grown from fresh patient isolates or frozen stock cultures using the appropriate cell line, medium, and incubation conditions suitable for the growth and culture of the particular virus. The following cells were used: (1) RMK (Rhesus monkey kidney) cells for influenza and parainfluenza viruses; (2) HEp-2 (human larynx carcinoma) cells for respiratory syncycial virus and adenovirus; and (3) VERC (African Green monkey kidney) cells for mumps. All cells were grown to near-confluent monolayers in tubes or flasks with minimum essential medium with 5-10 percent serum at 37°C. Virus infections were carried out in the appropriate cell lines with minimum essential medium without serum at 37°C.

Virus-infected cultures were monitored and confirmed by observing the appearance of characteristic cytopathic effects and by testing with immunofluorescence assays using virus-type specific monoclonal antibodies. The following characteristic cytopathic effects were observed for each of the viruses used as follows: (1) Influenza A and B: areas of large, irregularly shaped, granular, or vaculated cells with progressive degeneration of the cell monolayer; (2) Parainfluenza 1: small rounded cells throughout the cell monolayer; (3) Parainfluenza 2: dark, granular, and irregular syncytia which retract from the cell monolayer; (4) Parainfluenza 3: elongated, fusiform cells that eventually retract and pull away from the cell monolayer; (5) Respiratory syncytial virus: large, irregularly shaped syncytia that appear as large multinucleated cells with indistinct borders throughout the cell monolayer; (6) Adenovirus: large, rounded pycnotic cells which eventually aggregate into rounded cell sheets and detach from the culture vessel; and (7) Mumps: syncytia with vacuolation and cell degeneration throughout the cell monolayer. Commercial virus type-specific monoclonal antibodies were used in fluorescence assays to confirm growth of each virus in the inoculated cultures. Cells from the infected cultures were methanol fixed on glass slides or coverslips and then incubated at 37°C with fluorescein-labeled monoclonal antibodies. Both direct and indirect fluorescence assays were used. For the direct assays, the fixed, virus infected cells were incubated for 30 minutes with virus type-specific, fluorescein isothiocyanate (FITC) labeled monoclonal antibodies. For the indirect assays, the fixed, virus infected cells were incubated for 30 minutes with virus type-specific, unlabeled monoclonal antibodies, followed by incubation for an additional 30 minutes with secondary FITC-labeled monoclonal antibodies. The slides or coverslips were overlaid with mounting medium and examined using a fluorescent microscope. A positive virus result was verified by the presence of a characteristic, virus-specific, apple-green staining. The presence of progressive and characteristic cytopathic effects (along with accompanying specific virus confirmation with fluorescence assay) was an indication that the particular inoculated virus had proliferated maximally.

After achieving maximal virus growth and confirmation of the presence of the appropriate virus, the culture fluids were harvested for evaluation with the 4-methoxy-N-acetylneuraminic acid and 4,7-dimethoxy-N-acetylneuraminic acid chromogenic substrates. Virus-containing culture fluids (0.1 ml) were mixed with the chromogenic substrates in a 1 or 2 ml solution containing excipents and buffer designed to achieve optimal pH and neuraminidase activity. The buffer/excipient solution contained 35 mM malic/malate buffer with 10 mM calcium chloride, 0.85 percent sodium chloride, 0.1 percent mannitol, 0.5 percent methanol, and 0.1 percent methyl paraben. The substrate and buffer combination at a pH of about 5.4 was determined to provide specific detection of neuraminidase-producing viruses with clearly negative results for non-neuraminidase-producing viruses and virus-negative cultures.

The test solutions were incubated at 37°C for about one hour after which the reaction was stopped by the addition of 0.2 ml of a buffer concentrate which changed the pH to about 9 and helped increase the precipitation of the released chromogen. The completed reaction mixture was then transferred to a collection device containing a selectively porous membrane filter and an absorbent pad to wick away the solution whereby the precipitate is concentrated. The appearance of an appropriately colored precipitate indicated a positive reaction whereas the absence of such an appropriately colored precipitate indicated a negative reaction. Using the 5-bromo-3-indolyl chromogen, the characteristic color of the precipitate for a positive test was blue..

The following viruses were used to test the 5-bromo-3-indolyl-4-methoxy-N-acetylneuraminic acid substrate (BI-4-MeONeu5Ac) and the 5-bromo-3-indolyl-4, 7-dimethoxy-N-acetylneuraminic acid substrate (BI-4,7-(MeO)₂Neu5Ac).

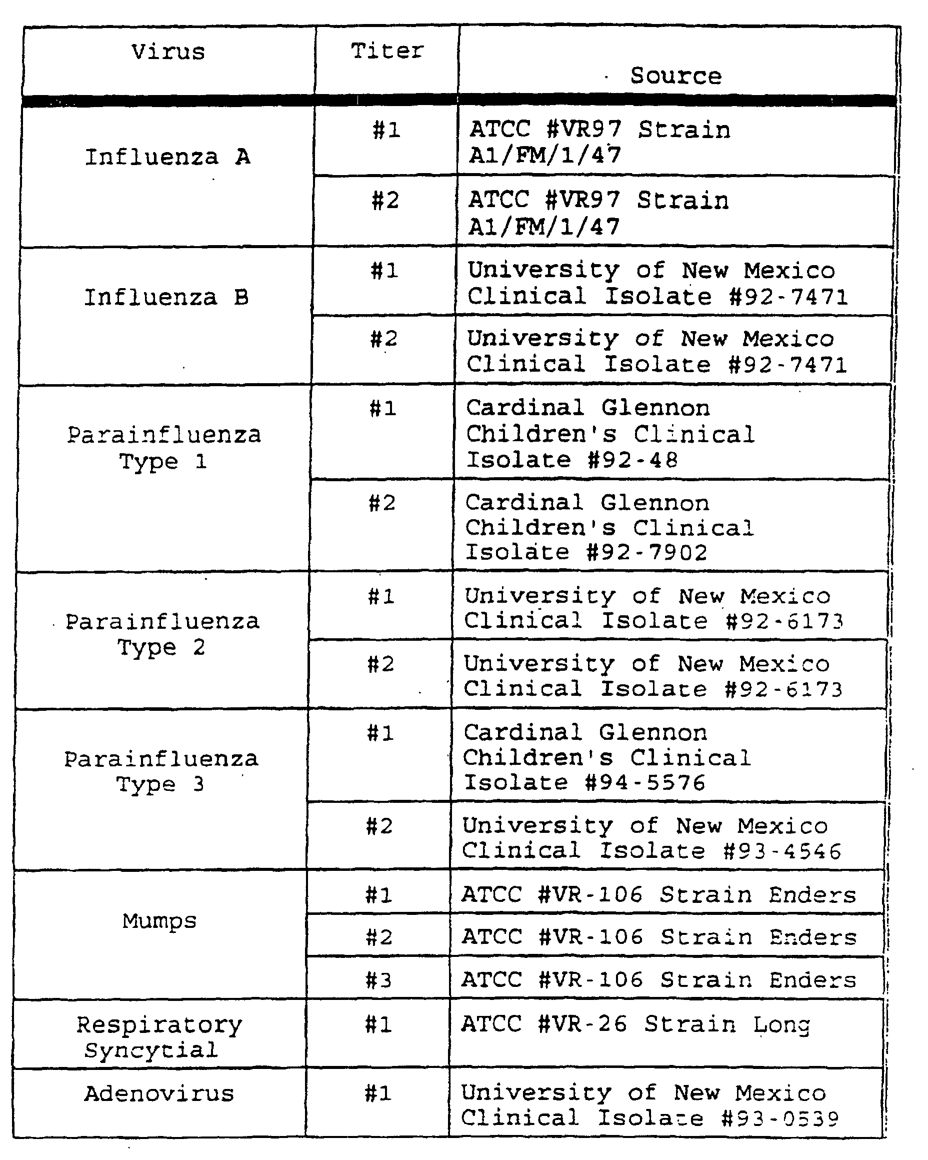

The following results were obtained using these various virus cultures. The virus cultures were grown to their maximal titers (as determined by observance of extensive -- about 90 to 100 percent -- virus-characteristic cytopathic effects in the cell sheet and confirmation with fluorescence assays of bright apple-green, virus-specific staining in the majority of cells) to insure that any reaction differences were not due to undetectable viral concentrations.

| Virus | Titer | BI-4-MeONeu5Ac | BI-4,7-(MeO)₂Neu5Ac |
|---|---|---|---|
| Influenza A | #1 | 1+ | 1+ |
| | #2 | 1+/2+ | 2+ |
| Influenza B | #1 | 1+ | 1+ |
| | #2 | 3+ | 3+ |
| Para-influenza Type 1 | #1 | negative | negative |
| | #2 | 2+ | negative |
| Para-influenza Type 2 | #1 | negative | negative |
| | #2 | 1+/2+ | negative |
| Para-influenza Type 3 | #1 | indeterminate | negative |
| | #2 | negative | negative |
| Mumps | #1 | negative | negative |
| | #2 | 1+ | negative |
| | #3 | 3+ | negative |
| Respiratory Syncytial | #1 | negative | negative |
| Adenovirus | #1 | negative | negative |

A racing scale of 1+ to 3+, based on the intensity of the reaction color, was used to indicate relative levels of detectable neuraminidase activity. The rating colors were matched to color samples from a standard reference source (The Pantone® Color Specific Book 747XR). A rating of 1+ (light color) indicates a low positive, 2+ (moderate color) indicates a moderate positive, and 3+ (deep color) indicates a high positive. A negative result is indicated by the absence of the characteristic color. An indeterminate result is indicated if a very faint trace or suggestion of the characteristic color may be present by visual observation.

As can be seen from these results, viruses containing influenza type A and B neuraminidase were the only tested organisms which gave a positive reaction with both the 4-methoxy-Neu5Ac and the 4,7-dimethoxy-Neu5Ac substrates undiluted and at all dilutions tested. For parainfluenza types 1, 2, and 3 and the mumps virus, the 4-mechoxy-Neu5Ac substrate gave both negative and positive reactions depending on the dilution. The 4,7-dimethoxy-Neu5Ac substrate gave a negative reaction with parainfluenza types 1, 2, and 3 and the mumps virus undiluted and at all dilutions. Non-neuraminidase containing viruses (i.e., respiratory syncytial virus and adenovirus) produced negative results with both substrates. Also, virus negative controls without virsu were appropriately negative. These results clearly demonstrate the specificity of 4,7-dialkoxy-N-acetylneuraminic acid chromogenic substrates for influenza type A and B viruses.

Example 4. Similar results as reported in Example 3 were obtained using 5-bromo-4-chloro-indol-3-ol and 4-methylumbelliferone as reporter molecules on 4-methoxy-Neu5Ac and 4,7-dimethoxy-Neu5Ac.

## Claims

1. A 4,7-dialkoxy-N-acetylneuraminic acid of the general formula 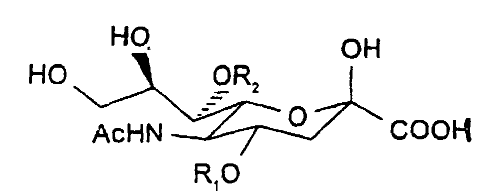 wherein R₁ and R₂ are alkyl groups containing 1 to 4 carbon atoms.

2. A 4,7-dialkoxy-N-acetylneuraminic acid as defined in claim 1, wherein both R₁ and R₂ are methyl groups.

3. A 4,7-dialkoxy-N-acetylneuraminic acid as defined in claim 1, wherein both R₁ and R₂ are ethyl groups.

4. A 4,7-dialkoxy-N-acetylneuraminic acid substrate of the general formula 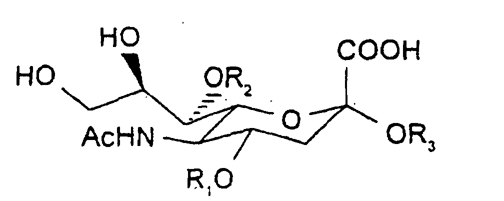 wherein R₁ and R₂ are alkyl groups containing 1 to 4 carbon atoms and R₃ is a chromogenic or fluorogenic group.

5. A 4,7-dialkoxy-N-acetylneuraminic acid substrate as defined in claim 4, wherein both R₁ and R₂ are methyl groups and R₃ is a chromogenic group.

6. A 4,7-dialkoxy-N-acetylneuraminic acid substrate as defined in claim 5, wherein R₃ is selected from the group consisting of 4-methylumbelliferyl, 3-cyanoumbelliferyl, 2-nitrophenyl, 4-nitrophenyl, 3-resorufin, 5-bromo-4-chloro-3-indolyl, 5-bromo-3-indolyl, 3-indolyl, nitrophenylazophenyl, nitrophenylazoresorcinyl, 3-methoxyphenyl, 3-dimethylaminophenyl, 4-chloro-1-naphthyl, and 6-bromo-2-naphthyl.

7. A 4,7-dialkoxy-N-acetylneuraminic acid substrate as defined in claim 6, wherein R₃ is 5-bromo-3-indolyl.

8. A 4,7-dialkoxy-N-acetylneuraminic acid substrate as defined in claim 4, wherein both R₁ and R₂ are ethyl groups and R₃ is a chromogenic group.

9. A 4,7-dialkoxy-N-acetylneuraminic acid substrate as defined in claim 8, wherein R₃ is selected from the group consisting of 4-methylumbelliferyl. 3-cyanoumbelliferyl, 2-nitrophenyl, 4-nitrophenyl, 3-resorufin, 5-bromo- 4-chloro-3-indolyl, 5-bromo-3-indolyl, 3-indolyl, nitrophenylazophenyl, nitrophenylazoresorcinyl, 3-methoxyphenyl, 3-dimethylaminophenyl, 4-chloro-1-naphthyl, and 6-bromo-2-naphthyl.

10. A method for detecting influenza type A and B viruses in a clinical sample from an individual suspected of having a respiratory viral infection, said method comprising:
(1) incubating the clinical sample with a chromogenic or fluorogenic 4,7-dialkoxy N-acetylneuraminic acid substrate of the general formula 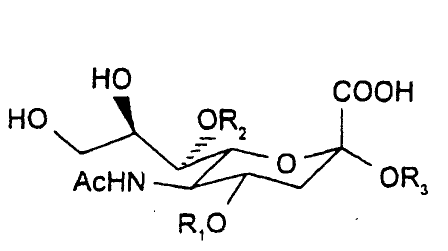 wherein R₁ and R₂ are alkyl radicals containing 1 to 4 carbon atoms and R₃ is a chromogenic or fluorogenic group that exhibits a distinct and characteristic color when cleaved from the substrate or a salt of the substrate;
(2) observing the incubated clinical sample to determine whether the distinct and characteristic color is formed, wherein the formation of the distinct and characteristic color indicates the presence of influenza type A or B viruses in the clinical sample.

11. A method as defined in claim 10, wherein both R₁ and R₂ are methyl groups and R₃ is a chromogenic group.

12. A method as defined in claim 11, wherein R₃ is selected from the group consisting of
4-methylumbelliferyl, 3-cyanoumbelliferyl, 2-nitrophenyl, 4-nitrophenyl, 3-resorufin, 5-bromo- 4-chloro-3-indolyl, 5-bromo-3-indolyl, 3-indolyl, nitrophenylazophenyl, nitrophenylazoresorcinyl, 3-methoxyphenyl, 3-dimethylaminophenyl, 4-chloro-1-naphthyl, and 6-bromo-2-naphthyl.

13. A method as defined in claim 12, wherein R₃ is 5-bromo-3-indolyl.

14. A method as defined in claim 10, wherein both R₁ and R₂ are ethyl groups and R₃ is a chromogenic group.

15. A method as defined in claim 14, wherein R₃ is selected from the group consisting of
4-methylumbelliferyl, 3-cyanoumbelliferyl, 2-nitrophenyl, 4-nitrophenyl, 3-resorufin, 5-bromo- 4-chloro-3-indolyl, 5-bromo-3-indolyl, 3-indolyl, nitrophenylazophenyl, nitrophenylazoresorcinyl, 3-methoxyphenyl, 3-dimethylaminophenyl, 4-chloro-1-naphthyl, and 6-bromo-2-naphthyl.

16. A method for detecting influenza type A and B viruses in a clinical sample from an individual suspected of having a respiratory viral infection, said method comprising:
(1) dividing the clinical sample into a first portion and a second portion;
(2) incubating the first portion with a chromogenic or fluorogenic 4,7-dialkoxy N-acetylneuraminic acid first substrate of the general formula 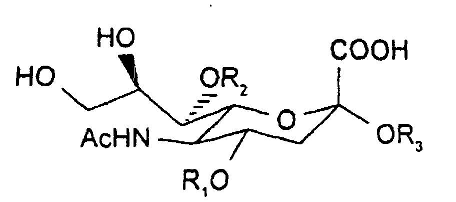 wherein R₁ and R₂ are alkyl radicals containing 1 to 4 carbon atoms and R₃ is a first chromogenic or fluorogenic group that exhibits a distinct and characteristic first color when cleaved from the first substrate or a salt of the first substrate;
(3) observing the incubated first portion to determine whether the distinct and characteristic first color is formed, wherein the formation of the distinct and characteristic first color indicates the presence of influenza type A or B viruses in the clinical sample;
(4) incubating the second portion with a chromogenic or fluorogenic 4-alkoxy N-acetylneuraminic acid second substrate of the general formula 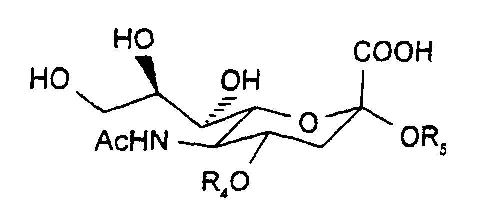 wherein R₄ is an alkyl radical containing 1 to 4 carbon atoms and R₅ is a second chromogenic or fluorogenic group that exhibits a distinct and characteristic second color when cleaved from the second substrate or a salt of the second substrate; and
(5) observing the incubated second portion to determine whether the distinct and characteristic second color is formed, wherein the formation of the distinct and characteristic second color indicates the presence of neuraminidase reactive viruses in the clinical sample;
wherein the presence of the first and second colors indicates the presence of influenza type A or B viruses alone or in combination with neuraminidase reactive viruses other than influenza type A and B viruses in the clinical sample;
wherein the presence of the second color and the absence of the first color indicates neuraminidase reactive viruses other than influenza type A and B viruses in the clinical sample; and
wherein the absence of the first and second colors indicates the absence of neuraminidase reactive viruses in the clinical sample.

17. A method as defined in claim 16, wherein both R₁ and R₂ are methyl groups and R₃ is a first chromogenic group.

18. A method as defined in claim 17, wherein R₄ is a methyl group and R₅ is a second chromogenic group.

19. A method as defined in claim 17, wherein R₃ and R₄ are independently selected from the group consisting of 4-methylumbelliferyl, 3-cyanoumbelliferyl, 2-nitrophenyl, 4-nitrophenyl, 3-resorufin, 5-bromo- 4-chloro-3-indolyl, 5-bromo-3-indolyl, 3-indolyl, nitrophenylazophenyl, nitrophenylazoresorcinyl, 3-methoxyphenyl, 3-dimethylaminophenyl, 4-chloro-1-naphthyl, and 6-bromo-2-naphthyl.

20. A method as defined in claim 19, wherein R₃ is 5-bromo-3-indolyl.

21. A method as defined in claim 18, wherein R₃ and R₄ are independently selected from the group consisting of 4-methylumbelliferyl, 3-cyanoumbelliferyl, 2-nitrophenyl, 4-nitrophenyl, 3-resorufin, 5-bromo- 4-chloro-3-indolyl, 5-bromo-3-indolyl, 3-indolyl, nicrophenylazophecyl, nitrophenylazoresorcinyl, 3-methoxyphenyl, 3-dimethylaminophenyl, 4-chloro-1-naphthyl, and 6-bromo-2-naphthyl.

## Patentansprüche

1. 4,7-Dialkoxy-N-acetylneuraminsäure der allgemeinen Formel: 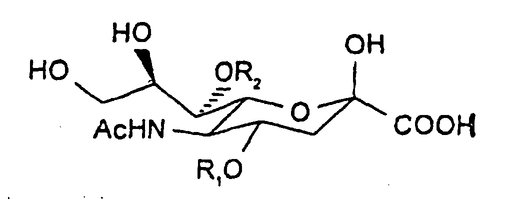 worin R₁ und R₂ Alkyl-Gruppen sind, die 1 bis 4 Kohlenstoffatome enthalten.

2. 4,7-Dialkoxy-N-acetylneuraminsäure gemäß Anspruch 1, worin sowohl R₁ als auch R₂ Methyl-Gruppen sind.

3. 4,7-Dialkoxy-N-acetylneuraminsäure gemäß Anspruch 1, worin sowohl R₁ als auch R₂ Ethyl-Gruppen sind.

4. 4,7-Dialkoxy-N-acetylneuraminsäure-Substrat der allgemeinen Formel: 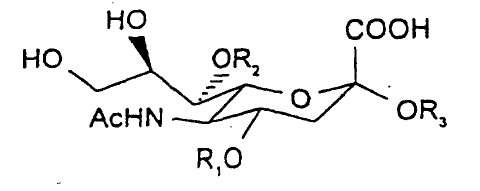 worin R₁ und R₂ Alkyl-Gruppen sind, die 1 bis 4 Kohlenstoffatome enthalten, und R₃ eine chromogene oder fluorogene Gruppe ist.

5. 4,7-Dialkoxy-N-acetylneuraminsäure-Substrat gemäß Anspruch 4, worin sowohl R₁ als auch R₂ Methyl-Gruppen sind und R₃ eine chromogene Gruppe ist.

6. 4,7-Dialkoxy-N-acetylneuraminsäure-Substrat gemäß Anspruch 5, worin R₃ ausgewählt ist aus der Gruppe bestehend aus 4-Methylumbelliferyl,
3-Cyanoumbelliferyl, 2-Nitrophenyl, 4-Nitrophenyl, 3-Resorufin, 5-Brom-4-chlor-3-indolyl, 5-Brom-3-indolyl, 3-Indolyl, Nitrophenylazophenyl, Nitrophenylazoresorcinyl, 3-Methoxyphenyl, 3-Dimethylaminophenyl, 4-Chlor-1-naphthyl und 6-Brom-2-naphthyl.

7. 4,7-Dialkoxy-N-acetylneuraminsäure-Substrat gemäß Anspruch 6, worin R₃ 5-Brom-3-indolyl ist.

8. 4,7-Dialkoxy-N-acetylneuraminsäure-Substrat gemäß Anspruch 4, worin sowohl R₁ als auch R₂ Ethyl-Gruppen sind und R₃ eine chromogene Gruppe ist.

9. 4,7-Dialkoxy-N-acetylneuraminsäure-Substrat gemäß Anspruch 8, worin R₃ ausgewählt ist aus der Gruppe bestehend aus 4-Methylumbelliferyl,
3-Cyanoumbelliferyl, 2-Nitrophenyl, 4-Nitrophenyl, 3-Resorufin, 5-Brom-4-chlor-3-indolyl, 5-Brom-3-indolyl, 3-Indolyl, Nitrophenylazophenyl, Nitrophenylazoresorcinyl, 3-Methoxyphenyl, 3-Dimethylaminophenyl, 4-Chlor-1-naphthyl und 6-Brom-2-naphthyl.

10. Verfahren zum Nachweis von Influenza-Viren vom Typ A und B in einer klinischen Probe von einer Einzelperson, die im Verdacht steht, eine virale Infektion der Atemwege zu haben, wobei das Verfahren umfaßt:
(1) Inkubieren der klinischen Probe mit einem chromogenen oder fluorogenen 4,7-Dialkoxy-N-acetylneuraminsäure-Substrat der allgemeinen Formel: 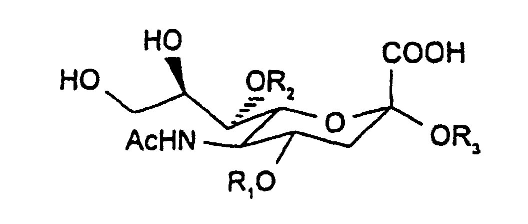 worin R₁ und R₂ Alkyl-Reste sind, die 1 bis 4 Kohlenstoffatome enthalten, und R₃ eine chromogene oder fluorogene Gruppe ist, die eine deutliche und charakteristische Farbe zeigt, wenn sie von dem Substrat oder einem Salz des Substrats abgespalten wird;
(2) Beobachten der inkubierten klinischen Probe, um zu bestimmen, ob die deutliche und charakteristische Farbe gebildet wird, wobei die Bildung der deutlichen und charakteristischen Farbe auf die Anwesenheit von Influenza-Viren vom Typ A oder B in der klinischen Probe hinweist.

11. Verfahren gemäß Anspruch 10, worin sowohl R₁ als auch R₂ Methyl-Gruppen sind und eine R₃ eine chromogene Gruppe ist.

12. Verfahren gemäß Anspruch 11, worin R₃ ausgewählt ist aus der Gruppe bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 5-Brom-3-indolyl, 3-Indolyl, Nitrophenylazophenyl, Nitrophenylazoresorcinyl, 3-Methoxyphenyl, 3-Dimethylaminophenyl, 4-Chlor-1-naphthyl und 6-Brom-2-naphthyl.

13. Verfahren gemäß Anspruch 12, worin R₃ 5-Brom-3-indolyl ist.

14. Verfahren gemäß Anspruch 10, worin sowohl R₁ als auch R₂ Ethyl-Gruppen sind und R₃ eine chromogene Gruppe ist.

15. Verfahren gemäß Anspruch 14, worin R₃ ausgewählt ist aus der Gruppe bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 5-Brom-3-indolyl, 3-Indolyl, Nitrophenylazophenyl, Nitrophenylazoresorcinyl, 3-Methoxyphenyl, 3-Dimethylaminophenyl, 4-Chlor-1-naphthyl und 6-Brom-2-naphthyl.

16. Verfahren zum Nachweis von Influenza-Viren vom Typ A und B in einer klinischen Probe von einer Einzelperson, die im Verdacht steht, eine virale Infektion der Atemwege zu haben, wobei das Verfahren umfaßt:
(1) Teilen der klinischen Probe in einen ersten Teil und einen zweiten Teil;
(2) Inkubieren des ersten Teils mit einem chromogenen oder fluorogenen ersten 4,7-Dialkoxy-N-acetylneuraminsäure-Substrat der allgemeinen Formel: 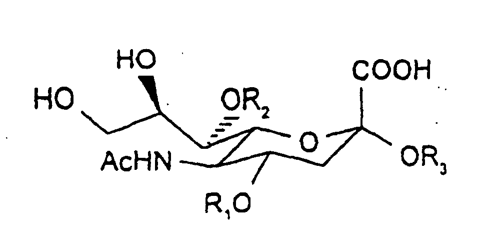 worin R₁ und R₂ Alkyl-Reste sind, die 1 bis 4 Kohlenstoffatome enthalten, und R₃ eine erste chromogene oder fluorogene Gruppe ist, die eine deutliche und charakteristische erste Farbe zeigt, wenn sie von dem ersten Substrat oder einem Salz des ersten Substrats abgespalten wird;
(3) Beobachten des inkubierten ersten Teils, um zu bestimmen, ob die deutliche und charakteristische erste Farbe gebildet wird, wobei die Bildung der deutlichen und charakteristischen ersten Farbe auf die Anwesenheit von Influenza-Viren vom Typ A oder B in der klinischen Probe hinweist;
(4) Inkubieren des zweiten Teils mit einem chromogenen oder fluorogenen zweiten 4-Alkoxy-N-acetylneuraminsäure-Substrat der allgemeinen Formel: 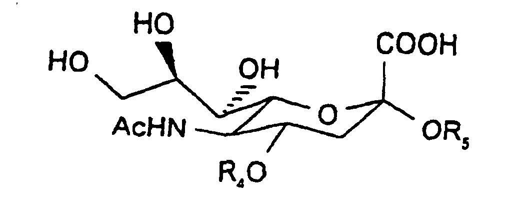 worin R₄ ein Alkyl-Rest ist, der 1 bis 4 Kohlenstoffatome enthält, und R₅ eine zweite chromogene oder fluorogene Gruppe ist, die eine deutliche und charakteristische zweite Farbe zeigt, wenn sie von dem zweiten Substrat oder einem Salz des zweiten Substrats abgespalten wird; und
(5) Beobachten des inkubierten zweiten Teils, um zu bestimmen, ob die deutliche und charakteristische zweite Farbe gebildet wird, wobei die Bildung der deutlichen und charakteristischen zweiten Farbe auf die Anwesenheit von Neuraminidase reaktiven Viren in der klinischen Probe hinweist;
wobei die Anwesenheit der ersten und zweiten Farbe auf die Anwesenheit von Influenza-Viren vom Typ A oder B allein oder in Kombination mit Neuraminidase reaktiven Viren, andere als Influenza-Viren vom Typ A und B, in der klinischen Probe hinweist;
wobei die Anwesenheit der zweiten Farbe und das Fehlen der ersten Farbe auf Neuraminidase reaktive Viren, andere als Influenza-Viren vom Typ A und B in der klinischen Probe hinweist; und
wobei die Abwesenheit der ersten und zweiten Farbe auf die Abwesenheit von Neuraminidase reaktiven Viren in der klinischen Probe hinweist.

17. Verfahren gemäß Anspruch 16, worin sowohl R₁ als auch R₂ Methyl-Gruppen sind und R₃ eine erste chromogene Gruppe ist.

18. Verfahren gemäß Anspruch 17, worin R₄ eine Methyl-Gruppe ist und R₅ eine zweite chromogene Gruppe ist.

19. Verfahren gemäß Anspruch 17, worin R₃ und R₄ unabhängig ausgewählt sind aus der Gruppe bestehend aus
4-Methylumbelliferyl, 3-Cyanoumbelliferyl, 2-Nitrophenyl, 4-Nitrophenyl, 3-Resorufin, 5-Brom-4-chlor-3-indolyl, 5-Brom-3-indolyl, 3-Indolyl, Nitrophenylazophenyl, Nitrophenylazoresorcinyl, 3-Methoxyphenyl, 3-Dimethylaminophenyl, 4-Chlor-1-naphthyl und 6-Brom-2-naphthyl.

20. Verfahren gemäß Anspruch 19, worin R₃ 5-Brom-3-indolyl ist.

21. Verfahren gemäß Anspruch 18, worin R₃ und R₄ unabhängig ausgewählt sind aus der Gruppe bestehend aus
4-Methylubelliferyl, 3-Cyanoumbelliferyl, 2-Nitrophenyl, 4-Nitrophenyl, 3-Resorufin, 5-Brom-4-chlor-3-indolyl, 5-Brom-3-indolyl, 3-Indolyl, Nitrophenylazophenyl, Nitrophenylazoresorcinyl, 3-Methoxyphenyl, 3-Dimethylaminophenyl, 4-Chlor-1-naphthyl und 6-Brom-2-naphthyl.

## Revendications

1. Acide 4,7-dialcoxy-N-acétylneuraminique de formule générale 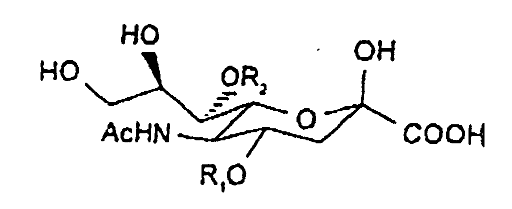 dans laquelle R₁ et R₂ représentent des groupes alkyle contenant 1 à 4 atomes de carbone.

2. Acide 4,7-dialcoxy-N-acétylneuraminique selon la revendication 1, où R₁ et R₂ représentent tous deux des groupes méthyle.

3. Acide 4,7-dialcoxy-N-acétylneuraminique selon la revendication 1, où R₁ et R₂ représentent tous deux des groupes éthyle.

4. Substrat d'acide 4,7-dialcoxy-N-acétylneuraminique de formule générale 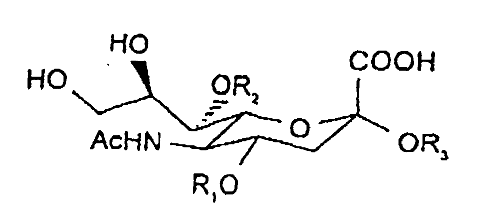 dans laquelle R₁ et R₂ représentent des groupes alkyle contenant 1 à 4 atomes de carbone et R₃ représente un groupe chromogène ou fluorogène.

5. Substrat d'acide 4,7-dialcoxy-N-acétylneuraminique selon la revendication 4, où R₁ et R₂ représentent tous deux des groupes méthyle et R₃ représente un groupe chromogène.

6. Substrat d'acide 4,7-dialcoxy-N-acétylneuraminique selon la revendication 5, où R₃ est choisi dans le groupe formé par les groupes 4-méthylumbelliféryle, 3-cyanoumbelliféryle, 2-niotrophényle, 4-nitrophényle, 3-résorufine, 5-bromo-4-chloro-3-indolyle, 5-bromo-3-indolyle, 3-indolyle, nitrophénylazophényle, nitrophénylazorésorcinyle, 3-méthoxyphényle, 3-diméthylaminophényle, 4-chloro-1-naphtyle, et 6-bromo-2-naphtyle.

7. Substrat d'acide 4,7-dialcoxy-N-acétylneuraminique selon la revendication 6, où R₃ représente le groupe 5-bromo-3-indolyle.

8. Substrat d'acide 4,7-dialcoxy-N-acétylneuraminique selon la revendication 4, où R₁ et R₂ représentent tous deux des groupes éthyle et R₃ représente un groupe chromogène.

9. Substrat d'acide 4,7-dialcoxy-N-acétylneuraminique selon la revendication 8, où R₃ est choisi dans le groupe formé par les groupes 4-méthylumbelliféryle, 3-cyanoumbelliféryle, 2-nitrophényle, 4-nitrophényle, 3-résorufine, 5-bromo-4-chloro-3-indolyle, 5-bromo-3-indolyle, 3-indolyle, nitrophénylazophényle, nitrophénylazorésorcinyle, 3-méthoxyphényle, 3-diméthylaminophényle, 4-chloro-1-naphtyle, et 6-bromo-2-naphtyle.

10. Procédé pour détecter des virus de types A et B de la grippe dans un échantillon clinique provenant d'un individu suspecté d'être atteint d'une infection à virus des voies respiratoires, ledit procédé consistant :
(1) à incuber l'échantillon clinique avec un substrat d'acide 4,7-dialcoxy-N-acétylneuraminique chromogène ou fluorogène de formule générale 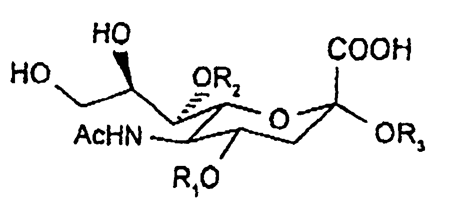 dans laquelle R₁ et R₂ représentent des radicaux alkyle contenant 1 à 4 atomes de carbone et R₃ représente un groupe chromogène ou fluorogène qui présente une couleur distincte et caractéristique quand il est coupé du substrat ou d'un sel du substrat ;
(2) à observer l'échantillon clinique incubé pour déterminer si oui ou non la couleur distincte et. caractéristique est formée, où la formation de la couleur distincte et caractéristique indique la présence de virus de types A et B de la grippe dans l'échantillon clinique.

11. Procédé selon la revendication 10, où R₁ et R₂ représentent tous deux des groupes méthyle et R₃ représente un groupe chromogène.

12. Procédé selon la revendication 11, où R₃ est choisi dans le groupe formé par les groupes 4-méthylumbelliféryle, 3-cyanoumbelliféryle, 2-nitrophényle, 4-nitrophényle, 3-résorufine, 5-bromo-4-chloro-3-indolyle, 5-bromo-3-indolyle, 3-indoyle, nitrophénylazophényle, nitrophénylazorésorcinyle, 3-méthoxyphényle, 3-diméthylaminophényle, 4-chlbro-1-naphtyle, et 6-bromo-2-naphtyle.

13. Procédé selon la revendication 12, où R₃ représente le groupe 5-bromo-3-indolyle.

14. Procédé selon la revendication 10, où R₁ et R₂ représentent tous deux des groupes éthyle et R₃ représente un groupe chromogène.

15. Procédé selon la revendication 14, où R₃ est choisi dans le groupe formé par les groupes 4-méthylumbelliféryle, 3-cyanoumbelliféryle, 2-nitrophényle, 4-nitrophényle, 3-résorufine, 5-bromo-4-chloro-3-indolyle, 5-bromo-3-indolyle, 3-indolyle, nitrophénylazophényle, nitrophénylazorésorcinyle, 3-méthoxyphényle, 3-diméthylaminophényle, 4-chloro-1-naphtyle, et 6-bromo-2-naphtyle.

16. Procédé pour détecter des virus de types A et B de la grippe dans un échantillon clinique provenant d'un individu suspecté d'être atteint d'une infection à virus des voies respiratoires, ledit procédé consistant :
(1) à partager l'échantillon clinique en une première portion et une seconde portion ;
(2) à incuber la première portion avec un premier substrat d'acide 4,7-dialcoxy-N-acétylneuraminique chromogène ou fluorogène de formule générale 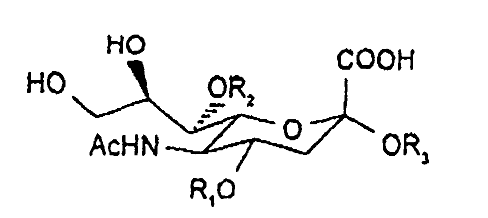 dans laquelle R₁ et R₂ représentent des radicaux alkyle contenant 1 à 4 atomes de carbone et R₃ représente un premier groupe chromogène ou fluorogène qui présente une première couleur distincte et caractéristique quand il est coupé du premier substrat ou d'un sel du premier substrat ;
(3) à observer la première portion incubée pour déterminer si oui ou non la première couleur distincte et caractéristique est formée, où la formation de la première couleur distincte et caractéristique indique la présence de virus de types A et B de la grippe dans l'échantillon clinique ;
(4) à incuber la seconde portion avec un second substrat d'acide 4-alcoxy-N-acétylneuraminique chromogène ou fluorogène de formule générale 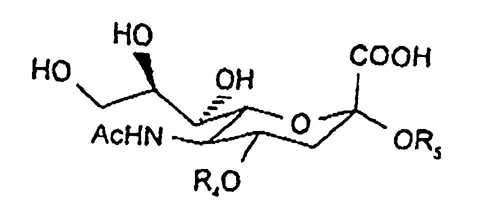 dans laquelle R₄ représente un radical alkyle contenant 1 à 4 atomes de carbone et R₅ représente un second groupe chromogène ou fluorogène qui présente une seconde couleur distincte et caractéristique quand il est coupé du second substrat ou d'un sel du second substrat ; et
(5) à observer la seconde portion incubée pour déterminer si oui ou non la seconde couleur distincte et caractéristique est formée, où la formation de la seconde couleur distincte et caractéristique indique la présence de virus réactifs à la neuraminidase dans l'échantillon clinique ;
où la présence des première et seconde couleurs indique la présence de virus de types A et B de la grippe seuls ou en combinaison avec des virus réactifs à la neuraminidase autres que les virus de types A et B de la grippe dans l'échantillon clinique ;
où la présence de la seconde couleur et l'absence de la première couleur indiquent la présence de virus réactifs à la neuraminidase autres que les virus de types A et B de la grippe dans l'échantillon clinique ; et
où l'absence des première et seconde couleurs indique l'absence de virus réactifs à la neuraminidase dans l'échantillon clinique.

17. Procédé selon la revendication 16, où R₁ et R₂ représentent tous deux des groupes méthyle et R₃ représente un premier groupe chromogène.

18. Procédé selon la revendication 17, où R₄ représente un groupe méthyle et R₅ représente un second groupe chromogène.

19. Procédé selon la revendication 17, où R₃ et R₄ sont, indépendamment l'un de l'autre, choisis dans le groupe formé par le groupes 4-méthylumbelliféryle, 3-cyanoumbelliféryle, 2-nitrophényle, 4-nitrophényle, 3-résorufine, 5-bromo-4-chloro-3-indolyle, 5-bromo-3-indolyle, 3-indolyle, nitrophénylazophényle, nitrophénylazorésorcinyle, 3-méthoxyphényle, 3-diméthylaminophényle, 4-chloro-1-naphtyle, et 6-bromo-2-naphtyle.

20. Procédé selon la revendication 19, où R₃ représente le groupe 5-bromo-3-indolyle.

21. Procédé selon la revendication 18, où R₃ et R₄ sont, indépendamment l'un de l'autre, choisis dans le groupe formé par les groupes 4-méthylumbelliféryle, 3-cyanoumbelliféryle, 2-nitrophényle, 4-nitrophényle, 3-résorufine, 5-bromo-4-chloro-3-indolyle, 5-bromo-3-indolyle, 3-indolyle, nitrophénylazophényle, nitrophénylazorésorcinyle, 3-méthoxyphényle, 3-diméthylaminophényle, 4-chloro-1-naphtyle, et 6-bromo-2-naphtyle.
